(19) Europäisches Patentamt
European Patent Office
Office européen des brevets

(11) EP 4 647 065 A1

(12) EUROPEAN PATENT APPLICATION

(43) Date of publication:
**12.11.2025 Bulletin 2025/46**

(21) Application number: **25174264.9**

(22) Date of filing: **05.05.2025**

(51) International Patent Classification (IPC):
***A61K 9/00*** *(2006.01)* ***A61K 9/107*** *(2006.01)*
***A61K 31/00*** *(2006.01)* ***A61P 17/04*** *(2006.01)*
***A61P 17/06*** *(2006.01)* ***A61K 47/10*** *(2017.01)*

(52) Cooperative Patent Classification (CPC):
**A61K 9/0014; A61K 9/107; A61K 31/00; A61K 47/10; A61P 17/04; A61P 17/06**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **09.05.2024 IT 202400010501**

(71) Applicant: **Laboratorio della Farmacia S.p.A.**
**30020 Quarto d'Altino VE (IT)**

(72) Inventors:
- **Fratter, Andrea**
  **30020 Quarto d'Altino VE (IT)**
- **Salvagnin, Marzio**
  **30020 Quarto d'Altino VE (IT)**
- **Ceretta, Giovanni**
  **30020 Quarto d'Altino VE (IT)**

# (54) VEHICLE FOR ACYLAMIDES BASED ON ALKYL ETHER DERIVATIVES OF POLYETHYLENE GLYCOL

(57) The present patent application relates to a carrier composition comprising a first solid active consisting of an acylamide of formula *I*, a dispersing agent consisting of a polyethylene glycol alkyl ether of formula *II,* wherein: $R_1$ is a linear or branched, saturated alkyl, characterized by a number of carbon atoms comprised between C1 and C4, wherein one of the carbons may or may not be bonded to a hydroxyl, *m* is comprised between 16 and 18, *n* is comprised between 7 and 10, z is comprised between 9 and 14, the first solid active and the dispersing agent are in a weight ratio comprised between 1:1.5 and 1:23, so that the first solid active is dispersed in the dispersing agent.

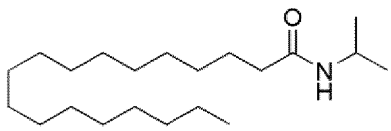

Figure 1

EP 4 647 065 A1

## Description

### FIELD OF THE INVENTION

The present invention falls into the technical field of technologies for delivering anti-inflammatory actives, such as acylamides, which are poorly soluble and/or difficult to incorporate within O/W or W/O emulsions.

### BACKGROUND ART

Acylamides (or AA) are substances chemically relatable to the medium-long chain fatty acid amides with primary aliphatic amines (Figure 1). They are of great pharmacological interest due to their cannabinoid-like activity.

From a pharmacodynamic point of view, the most investigated substance is Palmitoylethanolamide (or PEA), which cannot be considered to be a true endocannabinoid, since it lacks specificity for the CB1 and CB2 receptors, even if its interaction with *cannabinoid-like G-coupled receptors* GPR55 and GPR119 has been proven (Godlewski G. Prostaglandins & Other Lipid Mediators. 89 (3-4): 105-11). Its pharmacological action is achieved through binding to specific nuclear receptors, followed by a wide variety of biological functions related to chronic pain and inflammation. The main pharmacodynamic target appears to be PPAR-$\alpha$ receptors.

PEA is a biosimilar molecule of the endocannabinoid anandamide (Devane et al 1992); this similarity explains the pharmacological and clinical evidence of the interactions between PEA and the endogenous endocannabinoid system (Jonsson et al 2001; Petrosino et al 2010; Skaper and Di Marzo 2012).

Several molecules belonging to the acylamide family have been synthesized that have shown biological activities relatable to cannabinoids, although they lack receptor affinity and therefore lack the psychotropic effects of the latter and relatable to $\Delta$-9-tetrahydrocannabinol ($\Delta$-9-THC).

In particular, as described by the authors of EP1900365A2, it has been pointed out that the presence of an arachidyl radical (C20) is strictly related to a significant increase in the receptor affinity for the CB1 receptor and, therefore, to a psychoactive effect of the final molecule (Martin et al. 1999 ref. cit.). The authors of EP1900365A2 disclose that, despite the lack of the hydroxyl function in the amine used to synthesize the final acylamide, previously considered essential to exert the anti-inflammatory effect, it is possible to maintain this effect even in the absence of an interaction with the CB1 or CB2 receptors, thus introducing the hypothesis that there is a not yet identified receptor capable of mediating these effects at the tissue level.

In literature it is reported that molecules such as PEA, lacking receptor affinity for CB1 and CB2, act by both a local and systemic anti-inflammatory mechanism of action, especially in the context of neuropathic and osteo-articular pain, and also act as an analgesic agent in neuro- and osteo-inflammation [1-6].

#### *Prior art problem*

In view of the pharmacological relevance of acylamides, it is necessary to overcome some technological drawbacks related to the solubilization, stability and cutaneous application thereof in topical emulsifying formulations.

Acylamides are, indeed, usually difficult to incorporate/solubilize within the fatty phase of an emulsion, making thus difficult the topical application thereof.

For example, their solubilization within the fatty phase of an emulsion requires very high temperatures (for example, above 90°C); however, these temperatures are not compatible with most of the possible active substances and/or lipophilic excipients/diluents, as these may be thermally unstable at those temperatures, and thus degradable. This has an overall destabilizing effect on the emulsion to be obtained.

Moreover, acylamides are easily prone to recrystallization within the final emulsion after the latter has cooled down, a condition that further impairs the physico-chemical stability of the emulsion.

Last but not least, a partial recrystallization of the acylamides within the emulsion may affect the *trans*-cutaneous absorption thereof, reducing or compromising the biological activity thereof.

In the light of the prior art drawbacks, there exists the need to improve the solubility, stability and, therefore, biological activity of acylamides in solid form by obtaining a technological carrier that serves these purposes, and that can be included in topical formulations in the form of O/W or W/O emulsions.

### SUMMARY OF THE INVENTION

A first object of the invention is a *carrier composition* comprising

a first solid active consisting of an acylamide of formula *I*

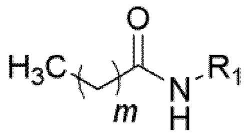

*formula I*

a dispersing agent consisting of a polyethylene glycol alkyl ether of formula *II*

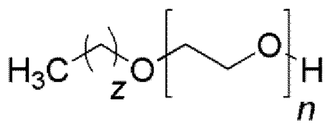

*formula II*

wherein

$R_1$ is a linear or branched, saturated alkyl, characterized by a number of carbon atoms comprised between C1 and C4, wherein one of the carbons may or may not be bonded to a hydroxyl,

*m* is comprised between 16 and 18,

*n* is comprised between 7 and 10,

*z* is comprised between 9 and 14,

the first solid active and the dispersing agent are in a weight ratio comprised between 1:1.5 and 1:23, so that the first solid active is dispersed in the dispersing agent.

A second object is a process for preparing the aforementioned carrier composition, comprising the following steps:

a) providing the first solid active and the dispersing agent,

b) heating the dispersing agent at a temperature ≥ 25°C and < 90°C to obtain the heated dispersing agent,

c) adding the first solid active into the heated dispersing agent to obtain a clear mixture,

f) letting the clear mixture cool to obtain the carrier composition.

Finally, a third object is a topical formulation, comprising

the aforementioned carrier composition,

suitable excipients and/or diluents,

*or*

the aforementioned carrier composition,

at least one second active, different from the first solid active and/or the aforementioned carrier composition, preferably the at least one second active being selected from the group consisting of: urea, panthenol, ammonium glycyrrhizate, ceramides, ammonium lactate, and combinations thereof,

suitable excipients and/or diluents,

wherein the topical formulation is an oil-in-water or water-in-oil emulsion.

***Advantages of the invention***

The invention allows to fulfill the needs of the background art in that the carrier composition allows the solid acylamide to be effectively dispersed within the dispersing agent. This improves the ease/versatility of use of acylamide, besides making the preparation of O/W or W/O emulsions less difficult.

In particular, the Applicant noted that the carrier composition allows an intimate relationship between the first solid active and the dispersing agent to be obtained; without being bound by any theory, the Applicant claims that a new physico-chemical entity is obtained. The first solid active loses its crystalline structure following hot solubilization in the dispersing agent, thus generating a molecular interaction between the two and leading to the formation of an amorphous form (see Example 5 and the related Figure 4). The amorphous form thus obtained also promotes the *trans*-cutaneous passage compared to a crystalline form.

A carrier composition in the form of a semi-solid mixture which is easily dispersible in the fatty phase of an emulsion is thus obtained (see Figures 2 and 3). In this form, the active acylamide proves to be much easier to incorporate than its natural solid form.

**DESCRIPTION OF THE FIGURES**

*Figure 1*: Chemical structure of StearoylIsoPropylAmide (or SIPA).

*Figure* 2: Acicular crystalline structure of Stearoylisopropylamide.

*Figure 3*: Appearance of the carrier composition obtained by hot dissolution and subsequent cooling of SIPA in Polydocanol (or POL).

*Figure 4*: *Overlapping* of *Differential Scanning Calorimetry* (or DSC) graphs relating to SIPA (active Stearoylisopropylamide; right) and SIPANOLO (carrier composition of the invention; bottom left), respectively.

*Figure 5:* Graph showing mean erythema index values starting from baseline values (T-1) as in the clinical study of Example 6.

*Figure 6:* Graph showing the mean % variation in the erythema index (compared to T0) observed after the application of the products P1427/A, P1429/A, P1442, as well as in the untreated area as in the clinical study of Example 6; in the lower box of the same Figure 6, the data of the erythema index normalized compared to T-1 (baseline skin values) are reported; these data are useful to highlight the variation in erythema induced by the SLS patch.

*Figure* 7: Graph showing the mean values of the stinging/burning sensation starting from *Tmax* (when the discomfort reaches its maximum peak, a few minutes after the application of the aqueous capsaicin solution) as in the clinical study of Example 6.

*Figure 8:* Digital image of the back of a volunteer (#11) after the application of the P1427/A formulation at the timepoints T0, T30min, T1h and T2h as in the clinical study of Example 6.

*Figure 9:* Digital image of the back of a volunteer (#18) after the application of the P1427/A formulation at the timepoints T0, T30min, T1h and T2h as in the clinical study of Example 6.

*Figure 10:* Digital image of the back of a volunteer (#19) after the application of the P1427/A formulation at the timepoints T0, T30min, T1h and T2h as in the clinical study of Example 6.

*Figure 11:* Digital image of the back of a volunteer (#02) after the application of the P1429/A formulation at the timepoints T0, T30min, T1h and T2h as in the clinical study of Example 6.

*Figure* 12: Digital image of the back of a volunteer (#13) after the application of the P1429/A formulation at the timepoints T0, T30min, T1h and T2h as in the clinical study of Example 6.

*Figure* 13: Digital image of the back of a volunteer (#15) after the application of the P1429/A formulation at the timepoints T0, T30min, T1h and T2h as in the clinical study of Example 6.

## DETAILED DESCRIPTION OF THE INVENTION

Hereinafter, the invention and the preferred embodiments thereof are described in more detail.

***Carrier composition of the invention.***

Carrier composition means a combination of ingredients able to act as a technological carrier for the first solid active consisting of an acylamide of formula *I*.

Dispersing agent means an ingredient which, when included in the carrier composition of the invention at a suitable weight ratio with respect to the first solid active, is able to effectively disperse the latter, which means that the first solid active is not prone to recrystallization and/or separation from the dispersing agent.

The first solid active and the dispersing agent included in the carrier composition will be described in more detail in the following.

*First solid active consisting of an acylamide of formula I*

Preferably, the first solid active consisting of acylamide of formula I is an anti-inflammatory and/or anti-itching active; it shows a mechanism of action overlapping that of PEA.

The first solid active consists of an acylamide of formula I

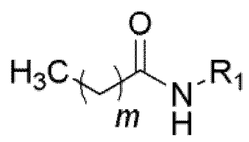

*formula I*

wherein

$R_1$ is a linear or branched, saturated alkyl, characterized by a number of carbon atoms comprised between C1 and C4, wherein one of the carbons may or may not be bonded to a hydroxyl,

*m* is comprised between 16 and 18, preferably is 16.

According to a first preferred embodiment, $R_1$ is a linear or branched, saturated alkyl, selected from the group consisting of: methyl, ethyl, propyl, isopropyl, butyl, isobutyl.

According to a second preferred embodiment, alternative to the first, $R_1$ is a linear, saturated alkyl characterized by a number of carbon atoms comprised between C1 and C2, wherein at least one carbon is bonded to a hydroxyl (-OH).

*First embodiment of the acylamide of formula I*

According to a first embodiment, $R_1$ is a linear or branched, saturated alkyl, selected from the group consisting of: methyl, ethyl, propyl, isopropyl, butyl, isobutyl.

Preferably, $R_1$ is isopropyl.

Preferably, *m* is comprised between 16 and 18, preferably is 16.

Preferably, the acylamide is the compound Stearoylisopropylamide of formula $I_A$ reported below.

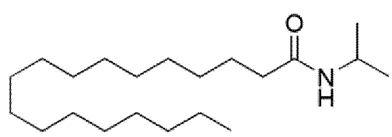

*formula $I_A$*

*Second embodiment of the acylamide of formula I*

According to a second embodiment, $R_1$ is a linear, saturated alkyl characterized by a number of carbon atoms comprised between C1 and C2, wherein at least one carbon is bonded to a hydroxyl (-OH).
Preferably, $R_1$ is ethyl and the second carbon (starting counting from the amide group) is bonded to a hydroxyl -OH.
Preferably, *m* is comprised between 16 and 18, preferably is 16.
Preferably, the acylamide is the compound Stearoylethanolamide of formula $I_B$ reported below.

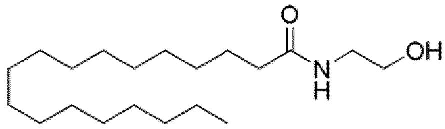

*formula $I_B$*

Preferably, the acylamide is in an amount comprised between 3% and 40% by weight, preferably between 4% and 35% by weight, preferably between 5% and 30% by weight, based on the total weight of the carrier composition.

*Dispersing agent*

Preferably, the dispersing agent is a cutaneous absorption promoter. Indeed, it should be noted that the dispersing agent, besides promoting the dispersion of the acylamide, is able to destructure the corneous-lipid matrix, thus promoting the increase in the trans-cutaneous flow of the acylamide.
Preferably, the dispersing agent is a non-ionic surfactant.
Preferably, the dispersing agent can also act as an active, preferably as an anesthetic.
The dispersing agent consists of a polyethylene glycol alkyl ether of formula *II*

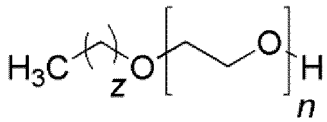

*formula II*

wherein

*n* is comprised between 7 and 10, preferably between 7 and 9, preferably between 7 and 8 or between 8 and 9, preferably is 9,

z is comprised between 9 and 14, preferably between 9 and 11, still preferably between 10 and 11, preferably is 11.

Preferably, the dispersing agent is the compound Polydocanol (also referred to as: laureth-9, or lauryl-macrogol-400, or lauryl alcohol ethoxylate) of the following formula $II_A$.

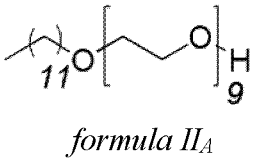

*formula $II_A$*

Preferably, the dispersing agent is in an amount comprised between 60% and 98% by weight, preferably between 65% and 98% by weight, preferably between 70% and 98% by weight, preferably between 70% and 95% by weight, based on the total weight of the carrier composition.

*Weight ratio of the first solid active to the dispersing agent*

Preferably, the first solid active and the dispersing agent are in a weight ratio comprised between 1:1.5 and 1:23,

preferably between 1:1.5 and 1:20, preferably between 1:2 and 1:20, preferably between 1:2 and 1:19, preferably between 1:2 and 1:15, preferably between 1:2 and 1:10, preferably between 1:2 and 1:9, preferably between 1:2 and 1:8, preferably between 1:2 and 1:6, preferably between 1:2 and 1:4, preferably of 1:4 or 1:9, or comprised between 1:4 and 1:9.

The weight ratio is such as to effectively promote the dispersion of the first solid active in the dispersing agent.

The carrier composition is in semi-solid form, the consistency thereof being similar to a waxy paste.

*Preferred embodiment of the carrier composition*

According to a particularly preferred embodiment, the carrier composition comprises

the first solid active consisting of the acylamide of formula $I_A$
the dispersing agent consisting of a polyethylene glycol alkyl ether of formula $II_A$

wherein
the first solid active and the dispersing agent are in a weight ratio comprised between 1:1.5 and 1:23, so that the first solid active is dispersed in the dispersing agent.

It should be noted that the specifications provided in the previous sections, such as relating to the weight ratios, also apply to this section about the preferred embodiment of the carrier composition.

***Process for preparing the carrier composition of the invention.***

The process for preparing the carrier composition comprises the following steps:

a) providing the first solid active and the dispersing agent,

b) heating the dispersing agent at a temperature ≥ 25°C and < 90°C, preferably ≥ 30°C and < 90°C, preferably ≥ 50°C and < 90°C, preferably ≥ 60°C and < 90°C, preferably ≥ 70°C and < 90°C, preferably ≥ 80°C and < 90°C, to obtain the heated dispersing agent,

c) adding the first solid active into the heated dispersing agent, preferably maintaining the temperature range as in step b) of heating the dispersing agent, to obtain a clear mixture,

f) letting the clear mixture cool, preferably to room temperature, to obtain the carrier composition.

Room temperature means a temperature comprised between 23°C and 28°C, preferably between 23°C and 25°C.

The Applicant noted that the hot solubilization of the solid acylamide in the dispersing agent produces, upon cooling, a matter having gelatinous consistency and white-opaque appearance, which is pourable and easy to incorporate into the fatty phases of emulsions at a lower temperature than the solid acylamide as is. In this form, the solid acylamide proves to be much easier to incorporate than its natural powder form, which requires to be dispersed in the fatty phase of the emulsion at very high temperatures (>90°C).

***Topical formulation comprising the carrier composition of the invention.***

The carrier composition can be included in a topical formulation.

Preferably, the topical formulation is in solid, semi-solid or liquid form.

Preferably, the topical formulation is in a technological form selected from: cream, salve, ointment, lotion.

According to a first preferred alternative, the topical formulation comprises

the carrier composition of the invention, preferably the carrier composition being the *sole* active included in the topical formulation,

suitable excipients and/or diluents,

wherein the topical formulation is an oil-in-water or water-in-oil emulsion.

According to a further preferred alternative, the topical formulation comprises

the carrier composition of the invention, preferably the carrier composition being one of the actives included in the

topical formulation,

at least one second active (hereinafter "second active"), different from the solid active consisting of an acylamide of formula *I*,

suitable excipients and/or diluents,

wherein the topical formulation is an oil-in-water or water-in-oil emulsion.

Suitable excipients and/or diluents are known to the person skilled in the art for preparing an oil-in-water or water-in-oil emulsion.

For example, suitable excipients and/or diluents can be selected from the group consisting of: emulsion stabilizers, film-forming agents, thickeners, preservatives, water, chelating agents, solubilizers, pH regulators, emulsifiers, antifoam agents, and combinations thereof.

Preferably, the second active is a cosmetic and/or pharmaceutical active ingredient, preferably it is selected from the group consisting of: skin conditioners, moisturizers, keratolytic agents, soothing agents, healing agents, skin regenerators, wetting agents, anti-inflammatory agents, anti-itching agents, anti-edema agents, anti-oxidant agents, chemical or physical sun filters, and combinations thereof.

Preferably, the second active is an active ingredient selected from the group consisting of: urea, allantoin, panthenol, ammonium glycyrrhizate, ammonium lactate, 18 β-glycyrrhetic acid, bisabolol, ceramides, cholesterol, saturated or unsaturated fatty acids, chemical or physical sun filters, and combinations thereof. Still preferably, the second active is an active ingredient selected from the group consisting of: urea, panthenol, ammonium glycyrrhizate, ceramides, ammonium lactate, and combinations thereof.

Preferably, the carrier composition is in an amount comprised between 1% and 5% by weight, preferably between 1% and 4% by weight, preferably between 1% and 3% by weight, preferably between 2% and 3% by weight, preferably of 2% or 3% by weight, based on the total weight of the formulation.

Preferably, the first solid active is in an amount comprised between 0.1% and 1% by weight, preferably between 0.2% and 0.8% by weight, preferably between 0.2% and 0.6% by weight, based on the total weight of the formulation.

Preferably, the dispersing agent is in an amount comprised between 1% and 3.5% by weight, preferably between 1% and 3% by weight, preferably between 1.5% and 2.8% by weight, preferably between 1.8% and 2.4% by weight, based on the total weight of the formulation.

Preferably, the carrier composition is incorporated into the fatty phase of the topical formulation in the form of an O/W or W/O emulsion, preferably is incorporated into the fatty phase at a temperature > 30°C and < 80°C.

Preferably, the carrier composition or the topical formulation is in the form of a cosmetic product or drug.

***Use of the carrier composition and the topical formulation comprising the carrier composition of the invention.***

Preferably, the carrier composition, or the topical formulation comprising the carrier composition, is for use in the treatment of skin inflammation, preferably it is for use in the treatment of itching and dermo-epidermal phlogosis.

Preferably, the carrier composition, or the topical formulation comprising the carrier composition, is for use in the treatment of symptoms associated with inflammatory skin conditions or dermo-epidermal phlogosis selected from the group consisting of: redness, itching, edema, pain, preferably acute pain.

Preferably, the carrier composition, or the topical formulation comprising the carrier composition, is for use as an anti-inflammatory or cutaneous local anesthetic.

It should be noted that the anti-inflammatory action exerted by the acylamide is assisted by the presence of the dispersing agent which acts as an active ingredient as well; preferably the dispersing agent can exert a local anesthetic action on the skin and mucous membranes by virtue of a mechanism inhibiting the passage of sodium in the voltage-gated channels of the sensory nerves [7].

Preferably, the carrier composition features a *"cannabinoid-like"* mechanism of action, which is of an anti-inflammatory type and linked to the interaction with the PPAR-α receptors and to the modulation of the release of pro-inflammatory cytokines, including TNF-α, Cytokines 1, 6, 17a; in addition, as mentioned before, the carrier composition shows a local anesthetic mechanism of action attributable to the inhibition of sodium current in the voltage-gated channels of the sensory nerves.

Preferably, the carrier composition, or the topical formulation comprising the carrier composition, is for use in the treatment of itching and/or erythema.

Preferably, the carrier composition, or the topical formulation comprising the carrier composition, is for use in the treatment of erythema being caused by or occurring in conjunction with: contact irritant dermatitis (or CID), and/or contact allergic dermatitis (or CAD), and/or atopic dermatitis (or AD), and/or psoriasis, and/or urticaria of various aetiology,

preferably urticaria of allergic aetiology, and/or solar erythema, and/or first-degree burns.

## EXAMPLES

***Example 1: Carrier composition of the invention.***

| Ingredient | Weight percent range based on the total weight of the composition (% w/w) |
|---|---|
| Stearoylisopropylamide (SIPA) | 5 - 30% |
| Polydocanol (POL) | 70 - 95% |

*Preparation method:*

Heat the POL at a temperature comprised between 80°C and 90°C, then dissolve the solid SIPA (which is in the form of little needles, as shown in Figure 2) therein, until a clear phase without undissolved aggregates is obtained.
The aforementioned clear phase is then let to cool to room temperature until a soft, white-opaque solid is obtained (as shown in Figure 3).

***Example 2: Topical formulation in the form of an O/W emulsion comprising the carrier composition of the invention.***

| Phase | Raw materials | % | Ingredients | Total % |
|---|---|---|---|---|
| A | PURIFIED WATER | 86.0000 | Aqua | 86.00000 |
| B | VASELINE OIL BFR 045 FU | 3.0000 | Paraffinum liquidum | 5.00000 |
| B | DERMOIL TG/CAPRYLIC CAPRIC TRIGLYCERIDE | 5.0000 | Caprylic/capric triglyceride | 3.00000 |
| A | ARISTOFLEX AVC | 0.4000 | Ammonium acryloyldimethyltaurate/VP copolymer | 2.00000 |
| B | SIPANOLO (LAURETH-9 90, SIPA 10) | 2.0000 | Laureth-9 | 1.80000 |
| | | | Stearoylisopropylamide | 0.20000 |
| B | SYMDIOL 68 | 0.8000 | 1,2-Hexanediol | 0.40000 |

| | | | | |
|---|---|---|---|---|
| | | | Caprylyl glycol | 0.40000 |
| B | SYMSAVE H | 0.5000 | Hydroxyacetophenone | 0.50000 |
| A | CHELATING AGENT GLDA | 0.3000 | Aqua | 0.15435 |
| | | | Tetrasodium glutamate diacetate | 0.14355 |
| | | | Sodium hydroxide | 0.00210 |
| A | XANTHAN GUM FEDCS PC | 0.3000 | Xanthan gum | 0.30000 |
| C | CITRIC ACID | 0.1000 | Citric acid | 0.10000 |
| | **Total** | **100.0000** | | **100.0000** |

*Preparation method:*

Prepare Phase A by bringing the water to 80°C, then dissolve the Chelating Agent GLDA. Next, proceed to hydrate the powder mixture consisting of Aristoflex AVC and Xanthan Gum under vigorous stirring, letting it to stir until complete and uniform gelation (check that no lumps or undissolved aggregates are present).

Prepare Phase B by melting all the components at 75°C until a clear or translucent liquid phase without undissolved aggregates is obtained.

Proceed to emulsify by pouring Phase B into Phase A under high mechanical stirring and continuing to stir for at least 5 minutes. Next, proceed to homogenize the system with a Silverson mixer at ¾ power for 3 minutes.

Proceed to cool the system up to 50°C and homogenize again with Silverson mixer at ½ power for 3 minutes. Let the system cool.

Finally, proceed to add citric acid (Phase C) up to pH=5.0.

*Use:* the O/W emulsion can be applied on the face and is recommended for atopic dermatitis.

***Example 3: Topical formulation in the form of a W/O emulsion comprising the carrier composition of the invention.***

| Phase | Raw materials | % | Ingredients | Total % |
|---|---|---|---|---|
| A | PURIFIED WATER | 43.3000 | Aqua | 43.30000 |
| B | VASELINE OIL BFR 045 FU | 36.0000 | Paraffinum liquidum | 36.00000 |
| A | GLYCERIN | 5.0000 | Glycerin | 4.975000 |
| | | | Aqua | 0.02500 |
| B | LAMEFORM TGI | 2.0000 | Polyglyceryl-3 diisostearate | 2.00000 |
| B | SHEA BUTTER/ CETIOL SB45 | 3.0000 | Butyrospermum parkii butter | 3.00000 |
| B | SASOLWAX 0907 | 1.70000 | Microcrystalline wax | 1.70000 |
| B | SIPANOLO | 3.0000 | Laureth-9 | 2.40000 |
| | | | Stearoylisopropylamide | 0.60000 |
| B | ELEMENT 14 PDMS 100 / XM PMX-200 SIL FLUID 100CS/ DIMETHICONE 100 | 3.0000 | Dimethicone | 3.00000 |

| | | | | |
|---|---|---|---|---|
| B | REFINED OLIVE OIL Ph.Eur. | 1.0000 | Olea europaea fruit oil | 1.00000 |
| A | SODIUM CHLORIDE | 0.4000 | Sodium chloride | 0.40000 |
| B | SENSIVA SC 50 | 0.5000 | Ethylhexylglycerin | 0.50000 |
| B | SYMSAVE H | 0.5000 | Hydroxyacetophenone | 0.50000 |
| A | CHELATING AGENT GLDA | 0.4000 | Aqua | 0.20580 |
| | | | Tetrasodium glutamate diacetate | 0.19140 |
| | | | Sodium hydroxide | 0.00280 |
| B | DL ALPHA TOCOPHEROL | 0.2000 | Tocopherol | 0.20000 |
| | **Total** | **100.0000** | | **100.0000** |

*Preparation method:*

Prepare Phase A by bringing the water to 80°C, then dissolve the Sodium chloride, Chelating Agent GLDA, and Glycerin.

Prepare Phase B by melting all the components at 75°C until a clear or translucent liquid phase without undissolved aggregates is obtained.

Proceed to emulsify by pouring Phase A into Phase B under high mechanical stirring and continuing to stir for at least 5 minutes. Next proceed to homogenize the system with a Silverson mixer at ¾ power for 5 minutes.

Proceed to cool the system up to 50°C and homogenize again with Silverson at ½ power for 5 minutes. Let the system cool.

*Use:* the W/O emulsion is recommended for very dry skin with epidermal phlogosis (psoriasis).

***Example 4: Topical formulation in the form of an O/W emulsion comprising the carrier composition of the invention.***

| Phase | Raw materials | % | Ingredients | Total % |
|---|---|---|---|---|
| A | PURIFIED WATER | 75.2000 | Aqua | 75.20000 |
| B | DERMOIL OP | 3.0000 | Ethylhexyl palmitate | 3.00000 |
| B | DERMOIL TG/CAPRYLIC CAPRIC TRIGLYCERIDE | 8.0000 | Caprylic capric triglyceride | 8.00000 |
| B | TEGO CARE 165 /SURF 165 | 2.0000 | Glyceryl stearate | 2.00000 |
| | | | PEG-100 stearate | 2.00000 |
| A | PROPYLENE GLYCOL FU-USP | 3.0000 | Propylene glycol | 3.00000 |
| B | GRIMED P 30/70 / SABONAL C1618 30/70 /CETYLSTEARYL ALCOHOL/TEGO ALKANOL 16 18 | 3.0000 | Cetearyl alcohol | 3.00000 |
| B | TEGOSOFT E BF | 2.0000 | PPG-15 stearyl ether | 1.99760 |
| | | | Pentaerythrityl tetra-di-t-butyl hydroxyhydrocinnamate | 0.00240 |
| B | SIPANOLO | 2.0000 | Laureth-9 | 1.80000 |

| | | | | |
|---|---|---|---|---|
| | | | Stearoylisopropylamide | 0.20000 |
| B | SYMDIOL 68 | 0.8000 | 1,2-Hexanediol | 0.40000 |
| | | | Caprylyl glycol | 0.40000 |
| B | SYMSAVE H | 0.5000 | Hydroxyacetophenone | 0.50000 |
| A | CHELATING AGENT GLDA | 0.3000 | Aqua | 0.15435 |
| | | | Tetrasodium glutamate diacetate | 0.14355 |
| | | | Sodium hydroxide | 0.00210 |
| B | DL ALPHA TOCOPHEROL | 0.2000 | Tocopherol | 0.20000 |
| | **Total** | **100.0000** | | **100.0000** |

*Preparation method:*

Prepare Phase A by bringing the water to 80°C, then dissolve the Chelating Agent GLDA and the Propylene Glycol.

Prepare Phase B by melting all the components at 75°C until a clear or translucent liquid phase without undissolved aggregates is obtained.

Proceed to emulsify by pouring Phase B into Phase A under high mechanical stirring and continuing to stir for at least 5 minutes. Next proceed to homogenize the system with a Silverson mixer at ¾ power for 3 minutes.

Proceed to cool the system up to 50°C and homogenize again with Silverson at ½ power for 3 minutes. Let the system cool.

*Use*: the O/W emulsion can be applied on the body.

***Example 5: Differential Scanning Calorimetry Analysis.***

The Applicant carried out *Differential Scanning Calorimetry* (or DSC) studies to evaluate how the structure of the compound Stearoylisopropylamide (or SIPA) and the carrier composition of the invention (referred to as SIPANOLO) changes, as outlined in Example 1.

As can be seen from Figure 4, the crystallization peak of Stearoylisopropylamide in the carrier composition of Example 1 disappears, which indicates the loss of the crystalline structure in favor of the amorphous one and of the consequent molecular interaction occurred between Stearoylisopropylamide and Polidocanol (or Laureth-9).

***Example 6: Clinical study for the placebo-controlled evaluation of the soothing effect of an active ingredient tested in two different concentrations.***

*6.1. Aim of the study*

The aim of the study is to evaluate the effect of an active ingredient (tested in two different concentrations) in

soothing both the skin alteration (*skin redness*) caused by an SLS (sodium lauryl sulphate) solution, and the skin discomfort (*stinging/burning sensation*) induced by a capsaicin solution.

To achieve this goal, a clinical study was conducted on 20 healthy subjects of both sexes, aged over 18 years, who tested positive to the prick test (the *Prick Test* is carried out by applying a 10% aqueous capsaicin solution on the skin rich in sensory innervation (hypersensitive skin) on both sides of the nasolabial fold, in order to detect the stinging/burning sensation that typically occurs in sensitive subjects within a few minutes).

6. *2. Test product*

*Product name*: P1427/A (0.2% SIPA, 1.8% Polydocanol); P1429/A (0.4% SIPA, 1.6% Polydocanol); P1442 (placebo).

*Way of use*:

To evaluate the soothing effect of the products on *skin redness*, 2 mg/cm$^2$ of products (actives and placebo) are applied, according to a standardized procedure, on three skin areas of the back of the volunteers, while a fourth skin site remains untreated and serves as a *control.* Treated and untreated areas are defined based on a previously defined randomization list.

To evaluate the soothing effect of the products on the *burning sensation*, the products are applied, using a soaked cotton bud, on the right/left nasolabial fold of each volunteer. This evaluation is carried out on two different days.

*6.3. Inclusion and exclusion criteria*

The subjects participating in the study were selected from a group of healthy subjects according to the following inclusion/non-inclusion criteria.

*6.3.1. Subjects meeting the inclusion criteria*

Twenty healthy subjects (males and females without any specific subdivision); Caucasian ethnicity; aged over 18; subjects who tested positive to the prick test; subjects who have not recently been involved in other similar studies; subjects enrolled in the National Health Service (NHS); subjects who certify the truth of the personal data communicated to the investigator; subjects able to understand the language used in the research centre and the information provided by the investigator; subjects able to comply with the instructions provided by the investigator and comply with the constraints and study-specific requirements; the pharmacological therapy (with the exception of the one envisaged by non-inclusion criteria) must have been stable for at least one month without any changes expected or planned during the study; willingness not to use products that may interfere with the test product; commitment not to change the daily routine or the lifestyle; subjects must have signed an informed consent form.

*6.3.2. Subjects meeting the exclusion criteria*

Subjects who do not meet the inclusion criteria; subjects suffering from acute or chronic diseases capable of interfering with the outcome of the study or that are considered hazardous to the subject or incompatible with the requirements of the study; subjects participating or planning to participate in other clinical studies; subjects deprived of liberty by administrative or legal decision or subject to guardianship; subjects unable to be contacted in an emergency; subjects admitted to a health or social care facility; subjects who have participated in a similar study without observing an adequate *washout* period; subjects suffering from an acute, chronic, or progressive disease that may interfere with the data of the study or that is considered by the investigator to be hazardous to the subject or incompatible with the study requirements; subjects on a pharmacological treatment that is incompatible with the study requirements; subjects suffering from a disease or skin condition that may interfere with the data of the study or that the investigator considers hazardous to the subject or incompatible with the study requirements; subjects who have shown allergies to cosmetic products, toiletries, drugs, patches, or cosmetic devices; pregnant or breastfeeding subjects.

Furthermore, a subject enrolled in the study may be withdrawn and considered a *dropout* when: (i) he/she no longer wishes to participate in the study, (ii) adverse reactions occur, which are judged to be serious and attributable to the tested product, (iii) the subject is no longer eligible to participate in the study, (iv) the subject develops a pathological condition, which is not related to the study but appears during the study period, (v) a concomitant treatment prescription is required, (vi) the study requirements are not met (significant deviation from the protocol), (vii) significant non-compliance with respect to the use of the product or the study protocol.

*6.4. Clinical study design*

*6.4.1. Evaluation of the soothing effect on skin alterations (**redness**) caused by an SLS (Sodium Lauryl Sulphate) solution, carried out on the back of the volunteers.*

The evaluation is carried out by selecting 4 skin areas on the lower back of each subject which are initially applied with a patch containing a 2% SLS aqueous solution in order to induce skin alterations (increased skin redness) (the induced skin alterations are transitory, not harmful to the health of the subjects and disappear spontaneously). The SLS solution is applied by Finn Chambers (large) onto Scanpor, a 12 mm diameter aluminum disc. The amount applied (50 $\mu$l) is sufficient to fill the chamber without the product overflowing when applied to the skin. The patches are left in contact with the skin surface for 24 hours.

15 minutes after the removal of the patches, the products (active and placebo formulations) are applied once by the investigator on the area of interest, in an amount of 2 $mg/cm^2$; a fourth area remains untreated and serves as a *control.* Treated and untreated (control) areas have been previously defined based on a randomization list.

The soothing effect of the products is evaluated 30 minutes, 1 hour and 2 hours after the application, as compared to placebo treated and untreated areas.

The evaluation is carried out according to the following scheme:

*Table 1*

| ***Experimental timepoints*** | ***Area 1 Control Area UNTREATED*** | ***Area 2 Treated area ACTIVE 1 (P1427/A)*** | ***Area 3 Treated area ACTIVE 2 (P1429/A)*** | ***Area 4 Treated area PLACEBO*** |
|---|---|---|---|---|
| **T-1** | Evaluation of the erythema index before the application of the SLS patch (baseline values).<br>Digital image acquisition of the back of the volunteers. | | | |
| | 2% SLS PATCH APPLICATION (the patches are left in contact with the skin surface for 24 hours) | | | |
| - | PATCH REMOVAL | | | |
| **T0** | Evaluation of the erythema index 15 minutes after the patch removal.<br>Digital image acquisition of the back of the volunteers. | | | |
| - | - | Application of the product by the investigator | | |
| **T30min*** | Evaluation of the erythema index 30 minutes after the application of the first products.<br>Digital image acquisition of the back of the volunteers. | | | |
| **T1h*** | Evaluation of the erythema index 1 hour after the application of the first products.<br>Digital image acquisition of the back of the volunteers. | | | |
| **T2h*** | Evaluation of the erythema index 2 hours after the first application of the product. | | | |
| | Digital image acquisition of the back of the volunteers. | | | |
| *The subjects remain in the laboratory in a temperature- and humidity-controlled room for 2 hours after the application of the product. | | | | |

*6.4.2. Evaluation of the soothing effect on the skin discomfort (**stinging/burning sensation**) induced by a capsaicin solution, evaluation carried out on the nasolabial folds of volunteers.*

The evaluation is carried out as follows:

***T0***: application of a 10% aqueous capsaicin solution on the skin on both sides of the nasolabial fold, in order to induce the stinging/burning sensation.

***Tmax***: when the discomfort reaches its maximum peak (a few minutes after the application of the 10% aqueous capsaicin solution), the investigator evaluates the intensity of the discomfort perceived and applies, by a soaked cotton bud, the tested active products on the right/left nasolabial fold according to a previously defined randomization list. The contralateral side is treated with the placebo/water formulation and serves as a control (this evaluation is carried out on two different days; on the first day the investigator applies the active 1 (P1427/A) and the placebo formulation (P1442) on the right/left nasolabial fold of the volunteer, and on the following day active 2 (P1429/A) and water are applied).

***T1st**:* the investigator reports the intensity of the discomfort perceived immediately after the first application of the products/water.

***T1'** / **T2'** / **T3'** / **T4'** / **T5'** / **T10'***: the investigator evaluates the intensity of the discomfort perceived at 1/2/3/4/5 and 10 minutes after the application of the products/water, respectively.

*6.5. Materials and methods*

The following sections report the materials and methods used in the study. All the study procedures are carried out under controlled temperature and humidity conditions (temperature 18-26°C and humidity 50 $\pm$ 10%). Before the visit, the subject undergoes an acclimatization period of 15-20 minutes under these conditions.

*6.5.1. PARAMETERS EVAL UATED ON THE BACK OF THE VOLOUNTEERS AT T-1/T0/T30min/T1h/T2h*

*Erythema Index*: burns, mechanical pressures, heat, chemical substances can induce the occurrence of skin redness (erythema).

The MEXAMETER 18 specifically measures the haemoglobin content (erythema) in the skin. The measurement is based on the principle of absorption. The special probe of the MEXAMETER 18 emits light of three defined wavelengths. A receiver measures the light reflected by the skin. The position of emitter and receiver ensures that only scattered and dispersed light is measured. Since the amount of the emitted light is defined, it is possible to calculate the amount of light absorbed by the skin. In order to measure the erythema, two different wavelengths are used to measure the absorbing capacity of the skin. One of these wavelengths corresponds to the spectral absorption peak of haemoglobin. The other wavelength was selected to avoid other chromatic influences (such as bilirubin). The results obtained are displayed on two clear digital displays on a scale from 0 to 999. It is important to highlight that there are no standard references for the value of erythema. The value of erythema is individual and sometimes it depends on the race or skin phototype. The probe is very sensitive and can discriminate small differences in colour.

The digital images of the back of the volunteers were taken using a digital reflex camera. The images of the best 6 cases (3 images for each active product) are reported in Figures 8-13.

*6.5.2. PARAMETER EVALUATED IN THE NASOLABIAL VOLUME AT Tmax/T1st/T1'/T2'/T3'/T4'/T5'/T10'*

*Clinical scoring of the stinging/burning sensation*: at each timepoint monitored, the investigator records, with the collaboration of the subject, the intensity of the perceived sensation according to the scores in the table.

*Table 2*

| *Clinical scoring of the stinging/burning sensation* | *Score* |
|---|---|
| No reaction | 1 |
| Mild reaction | 2 |
| Moderate reaction | 3 |
| Severe reaction | 4 |

*6.6. Statistical measuring methods*

The results are reported in the Tables in the respective units.

1) The mean values are calculated as: $m = \frac{\sum_1^n p}{n}$ where: *p* is the value of the parameter to be analyzed.

2) The standard error of the mean is calculated as:

$$SE = \frac{\sqrt{\frac{\sum_1^n (p_i^2) - \frac{\sum_1^n p_i^2}{n}}{(n-1)}}}{\sqrt{n}}$$

where: *p* is the value of the parameter to be analyzed, *n* is the number of subjects.

3) The mean percentage variations were calculated as:

$$\overline{\mathrm{Var}(\%)} = \sum_1^n \frac{X_T - X_0}{X_0}$$

wherein: $X_0$ is the value of the parameter to be analyzed at T0 or T-1;
$X_T$ is the value of the parameter to be analyzed at further experimental timepoints monitored.

All calculations were performed using a Microsoft® Excel worksheet.

The instrumental data (erythema index) are subjected to the two-way Student's t test. The intragroup statistical analysis is carried out on raw data recorded at each *timepoint* (T30min, T1h, T2h, T24h) compared to baseline (T-1) and compared to T0 (after the SLS patch removal). The intergroup statistical analysis was carried out on the % variations obtained in the treated areas compared to the control ones at each *timepoint.*

The clinical data (score of the stinging/burning sensation) were subjected to the signed-rank Wilcoxon test. The intragroup statistical analysis was carried out compared to *Tmax.* The intergroup statistical analysis was carried out on raw data relating to the treated area compared to the control one, at each experimental timepoint monitored. The statistical software used for this statistical analysis is NCSS 10.

Variations are considered statistically significant when the *p* values is ≤ 0.05.

The intragroup (*vs. T0)* or intergroup (e.g., active vs. placebo) statistical analysis criterium to reject the null hypothesis (no effect of the product) is set at $p<0.05$. For clinical evaluations, the positive effect of the product on the evaluated parameter is confirmed if an improvement in more than 50% of the subjects is recorded. Finally, for self-assessment questionnaires, product performance and pleasantness must be perceived by at least 60% of the subjects. When reference values or threshold values exist, these are used to validate the product claims.

The study start date was August 28, 2023, while the study end date was October 6, 2023.

*6.6 Results*

*6.6.1. Results of the soothing effect on SLS-induced skin erythema*

*Table 3*

| *Experimental timepoints* | |
|---|---|
| T-1 | Evaluation before the SLS patch application. |
| SLS PATCH APPLICATION | |
| T0 | Patches are removed after 24 hours. Evaluation after the patch removal. |
| PRODUCT APPLICATION ON THE AREA OF INTEREST | |
| T30min/T1h/T2h | Evaluation at 30 minutes, 1/2 hours after product application |

The graph in Figure 5 reports erythema index raw data expressed as Mexameter® arbitrary units ("a.u.").

The graph in Figure 6 reports the % variations in the erythema index values at each experimental timepoint after the application of the product. In the lower box in the Figure 6, the data of the erythema index normalized compared to T-1 (skin baseline values) are reported; these data are useful to highlight the variation in the erythema induced by the SLS patch.

In the Table below, the results (p values) of the intragroup and intergroup statistical analysis are reported. Statistically significant data (p<0.05) are highlighted in bold.

*Table 4*

| *INTRAGROUP STATISTICAL ANALYSIS (on raw data vs. T0)* | | | |
|---|---|---|---|
| | *T30min* | *T1h* | *T2h* |
| P1427/A | **0.001** | **0.000** | **0.006** |
| P1429/A | **0.029** | **0.001** | **0.023** |

(continued)

| INTRAGROUP STATISTICAL ANALYSIS (on raw data vs. T0) | | | |
|---|---|---|---|
| | *T30min* | *T1h* | *T2h* |
| P1442 (placebo) | 0.324 | 0.495 | 0.563 |
| Untreated (control) | 0.483 | 1.000 | 0.648 |
| | | | |
| *INTERGROUP STATISTICAL ANALYSIS (on % variations compared to T0)* | | | |
| | *T30min* | *T1h* | *T2h* |
| P 1427/A *vs.* P1429/A | 0.285 | 0.543 | 0.841 |
| P1427/A *vs.* P1442 | **0.031** | **0.002** | **0.003** |
| P1427/A vs. control | **0.002** | **0.000** | **0.003** |
| P1429/A *vs.* P1442 | 0.163 | **0.012** | **0.004** |
| P1429/A vs. control | **0.009** | **0.002** | **0.005** |
| P1442 vs. control | **0.049** | 0.143 | 0.712 |

As can be seen, in the skin site treated with P1427/A, a statistically significant decrease in SLS-induced skin erythema is observed at each *timepoint* (intragroup statistical analysis vs. T0), of -22.0% at T30min, of -33.6% at T1h and of -28.0% at T2h, respectively (see Figure 6). Furthermore, the results obtained with the application of P1427/A are significantly greater than those observed both at the skin site treated with P1442 and in the untreated one (control), at each *timepoint* (intergroup statistical analysis).

A statistically significant decrease in SLS-induced skin erythema is also observed at each *timepoint* (intergroup statistical analysis compared to T0) at the skin site treated with P1429/A, of -15.7% at T30min, of -30.2% at T1h and of -29.1% at T2h, respectively (see Figure 6). Furthermore, the results obtained with the application of P1429/A are significantly greater than those observed at the untreated skin site (control) at each *timepoint* and at the skin site treated with P1442 starting from T1h (intergroup statistical analysis).

No significant variations in the skin erythema are observed at the skin site treated with the P1442 formulation (placebo) and in the untreated area (control), at each *timepoint.* The results obtained with the application of P1442 are significantly greater than those observed at the untreated skin site (control) at T30min; however, this significance is no longer observed at the next *timepoint.*

Based on the results reported above, it can be concluded that the tested products P1427/A and P1429/A are effective in soothing the erythematous skin reaction (increased skin redness) induced by the SLS patch; no significant differences between the two formulations are observed.

Also consider Figures 8-13 relating to digital images of the back of different volunteers after the application of the P1427/A formulation and the P1429/A formulation at the timepoints T0, T30min, T1h and T2h.

*6.6.2. Results of the soothing effect on the capsaicin-induced stinging sensation*

Figure 7 shows the graph reporting the mean values of the stinging/burning sensation starting from *Tmax* (when the discomfort reaches its maximum peak, a few minutes after the application of the aqueous capsaicin solution).

In the Table below, the results (p values) of the intragroup and intergroup statistical analysis are reported. Statistically significant data ($p<0.05$) are highlighted in bold.

*Table 5*

| INTRAGROUP STATISTICAL ANALYSIS | | | | | | | |
|---|---|---|---|---|---|---|---|
| | *T1st* | *T1'* | *T2'* | *T3'* | *T4'* | *T5'* | *T10'* |
| P1427/A | 0.002 | 0.002 | 0.000 | 0.000 | 0.000 | 0.000 | 0.000 |
| P1429/A | 0.001 | 0.006 | 0.010 | 0.006 | 0.001 | 0.000 | 0.000 |
| P1442 (placebo) | 0.006 | 0.049 | 0.023 | 0.002 | 0.001 | 0.000 | 0.000 |

(continued)

| INTRAGROUP STATISTICAL ANALYSIS | | | | | | | |
|---|---|---|---|---|---|---|---|
| | *T1st* | *T1'* | *T2'* | *T3'* | *T4'* | *T5'* | *T10'* |
| Untreated (control) | 0.005 | 0.019 | 0.034 | 0.006 | 0.000 | 0.000 | 0.000 |
| | | | | | | | |
| *INTERGROUP STATISTICAL ANALYSIS* | | | | | | | |
| | *T1st* | *T1'* | *T2'* | *T3'* | *T4'* | *T5'* | *T10'* |
| P 1427/A *vs.* P1429/A | 1.000 | 0.857 | 0.072 | 0.082 | 0.054 | 0.290 | **0.046** |
| P1427/A *vs.* P1442 | 0.157 | 0.054 | **0.004** | **0.011** | **0.004** | **0.034** | 0.083 |
| P1427/A *vs.* control | 0.220 | 0.223 | **0.006** | **0.015** | **0.005** | 0.239 | 0.098 |
| P1429/A *vs.* P1442 | 0.406 | 0.084 | 0.289 | 0.386 | 0.175 | 0.331 | 0.620 |
| P1429/A *vs.* control | 0.123 | 0.220 | 0.071 | 0.119 | 0.054 | 0.557 | 0.710 |
| P1442 *vs.* control | 0.612 | 0.932 | 0.635 | 0.444 | 0.230 | 1.000 | 0.414 |

As can be seen, a statistically significant decrease in the stinging sensation induced by the capsaicin solution was observed in all the investigated areas starting from *T1st* (immediately after the application of the product/water) and at each next timepoint monitored.

The intergroup statistical analysis points out that the burning sensation still perceived at *T10'* is lower with the application of P1427/A (mean score 1.3) compared to P1429/A (mean score 1.6) (in this regard see also Figure 7).

Furthermore, the decrease in the stinging sensation observed with the application of P1427/A is faster than the one observed with the application of P1442 (starting from *T2'* up to *T5'*) and in the control area treated with water (starting from *T2'* up to *T4'*).

*6.7 Conclusions*

Based on the results, it can be concluded that the formulations or products P1427/A and P1429/A are effective in soothing the erythematous skin reaction (increased skin redness) induced by the SLS patch; no significant differences between the two formulations are observed.

As far as the capsaicin-induced stinging/burning sensation is concerned, a faster decrease is observed with the application of P1427/A compared to P1442 and to the application of water. Furthermore, the burning sensation still perceived at *T10*' is lower with the application of P1427/A compared to P1429/A.

**LITERATURE**

1. Lang-Illievich K, Klivinyi C, Rumpold-Seitlinger G, Dorn C, Bornemann-Cimenti H. The Effect of Palmitoylethanolamide on Pain Intensity, Central and Peripheral Sensitization, and Pain Modulation in Healthy Volunteers-A Randomized, Double-Blinded, Placebo-Controlled Crossover Trial. Nutrients. 2022 Oct 1;14(19):4084. doi: 10.3390/nu14194084.

2. Lang-Illievich K, Klivinyi C, Lasser C, Brenna CTA, Szilagyi IS, Bornemann-Cimenti H. Palmitoylethanolamide in the Treatment of Chronic Pain: A Systematic Review and Meta-Analysis of Double-Blind Randomized Controlled Trials. Nutrients. 2023 Mar 10;15(6):1350. doi: 10.3390/nu15061350.

3. Orefice NS, Alhouayek M, Carotenuto A, Montella S, Barbato F, Comelli A, Calignano A, Muccioli GG, Orefice G. Oral Palmitoylethanolamide Treatment Is Associated with Reduced Cutaneous Adverse Effects of Interferon-β1a and Circulating Proinflammatory Cytokines in Relapsing-Remitting Multiple Sclerosis. Neurotherapeutics. 2016 Apr;13(2):428-38. doi: 10.1007/s13311-016-0420-z.

4. Davis MP, Behm B, Mehta Z, Fernandez C. The Potential Benefits of Palmitoylethanolamide in Palliation: A Qualitative Systematic Review. American Journal of Hospice and Palliative Medicine®. 2019;36(12):1134-1154.

doi:10.1177/1049909119850807

5. Alhouayek M, Muccioli GG. Harnessing the anti-inflammatory potential of palmitoylethanolamide. Drug Discov Today. 2014 Oct;19(10):1632-9. doi: 10.1016/j.drudis.2014.06.007.

6. Hoareau L, Buyse M, Festy F, Ravanan P, Gonthier MP, Matias I, Petrosino S, Tallet F, d'Hellencourt CL, Cesari M, Di Marzo V, Roche R. Anti-inflammatory effect of palmitoylethanolamide on human adipocytes. Obesity (Silver Spring). 2009 Mar;17(3):431-8. doi: 10.1038/oby.2008.591.

7. Evans M et al. Polidocanol Inhibits Voltage-Gated Sodium Currents. Clin Dermatol Res J Vol: 5 Issue: 3.

8. Som I, Bhatia K, Yasir M. Status of surfactants as penetration enhancers in transdermal drug delivery. J Pharm Bioallied Sci. 2012 Jan;4(1):2-9. doi: 10.4103/0975-7406.92724.

## Claims

**1.** A carrier composition, comprising

a first solid active consisting of an acylamide of formula (*I*)

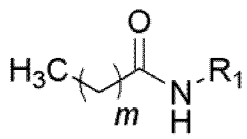

*formula (I)*

a dispersing agent consisting of a polyethylene glycol alkyl ether of formula (*II*)

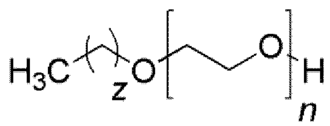

*formula (II)*

wherein
$R_1$ is a linear or branched, saturated alkyl, **characterized by** a number of carbon atoms comprised between C1 and C4, wherein one of the carbons may or may not be bonded to a hydroxyl,
*m* is comprised between 16 and 18,
*n* is comprised between 7 and 10,
*z* is comprised between 9 and 14,
the first solid active and the dispersing agent are in a weight ratio comprised between 1:1.5 and 1:23, so that the first solid active is dispersed in the dispersing agent.

**2.** The composition according to claim 1, wherein $R_1$ is a linear or branched, saturated alkyl, selected from the group consisting of: methyl, ethyl, propyl, isopropyl, butyl, isobutyl.

**3.** The composition according to claim 1 or 2, wherein $R_1$ is a linear, saturated alkyl **characterized by** a number of carbon atoms comprised between C1 and C2, wherein at least one carbon is bonded to a hydroxyl.

**4.** The composition according to any one of claims from 1 to 3, wherein *m* is 16.

**5.** The composition according to any one of claims from 1 to 4, wherein z is comprised between 9 and 11.

**6.** A process for preparing the carrier composition according to any one of claims from 1 to 5, comprising the following

steps:

a) providing the first solid active and the dispersing agent,
b) heating the dispersing agent at a temperature ≥ 25°C and < 90°C to obtain the heated dispersing agent,
c) adding the first solid active into the heated dispersing agent to obtain a clear mixture,
f) letting the clear mixture cool to obtain the carrier composition.

**7.** A topical formulation, comprising

the carrier composition according to any one of claims from 1 to 5,
suitable excipients and/or diluents,
*or*
the carrier composition according to any one of claims from 1 to 5,
at least one second active, different from the first solid active and/or the carrier composition, preferably the at least one second active being selected from the group consisting of: urea, panthenol, ammonium glycyrrhizate, ceramides, ammonium lactate, and combinations thereof,
suitable excipients and/or diluents,
wherein the topical formulation is an oil-in-water or water-in-oil emulsion.

**8.** The topical formulation according to claim 7, wherein the carrier composition is in an amount comprised between 1% and 5% by weight based on the total weight of the formulation.

**9.** The topical formulation according to any one of claims from 7 to 9, for use in the treatment of inflammatory skin conditions.

**10.** The topical formulation according to any one of claims from 7 to 9, for use in the treatment of itching and/or erythema, preferably the erythema being caused by or occurring in conjunction with: contact irritant dermatitis, and/or contact allergic dermatitis, and/or atopic dermatitis, and/or psoriasis, and/or urticaria of various aetiology, and/or solar erythema, and/or first-degree burns.

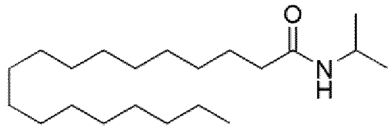

Figure 1

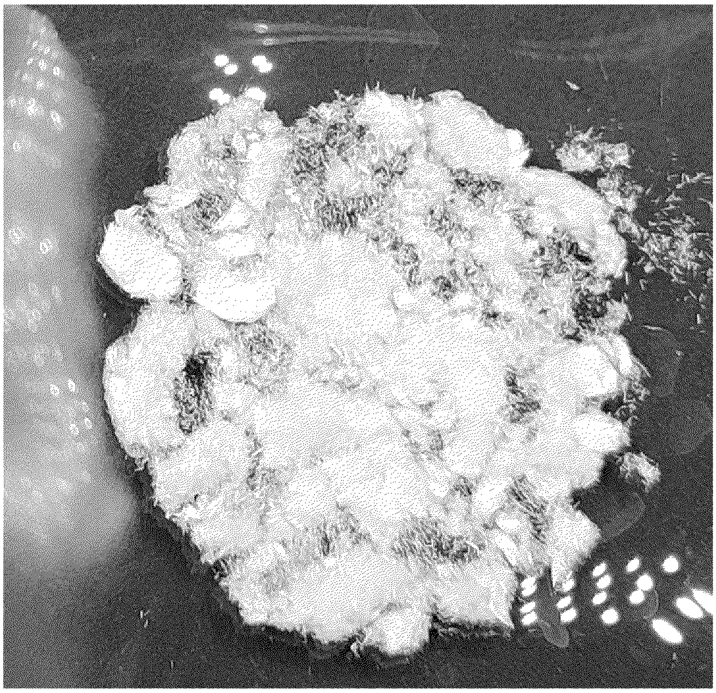

Figure 2

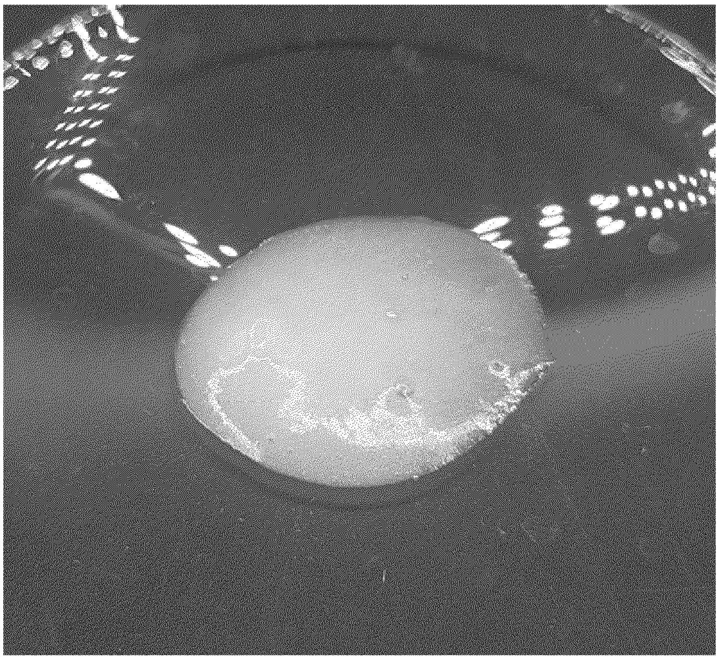

Figure 3

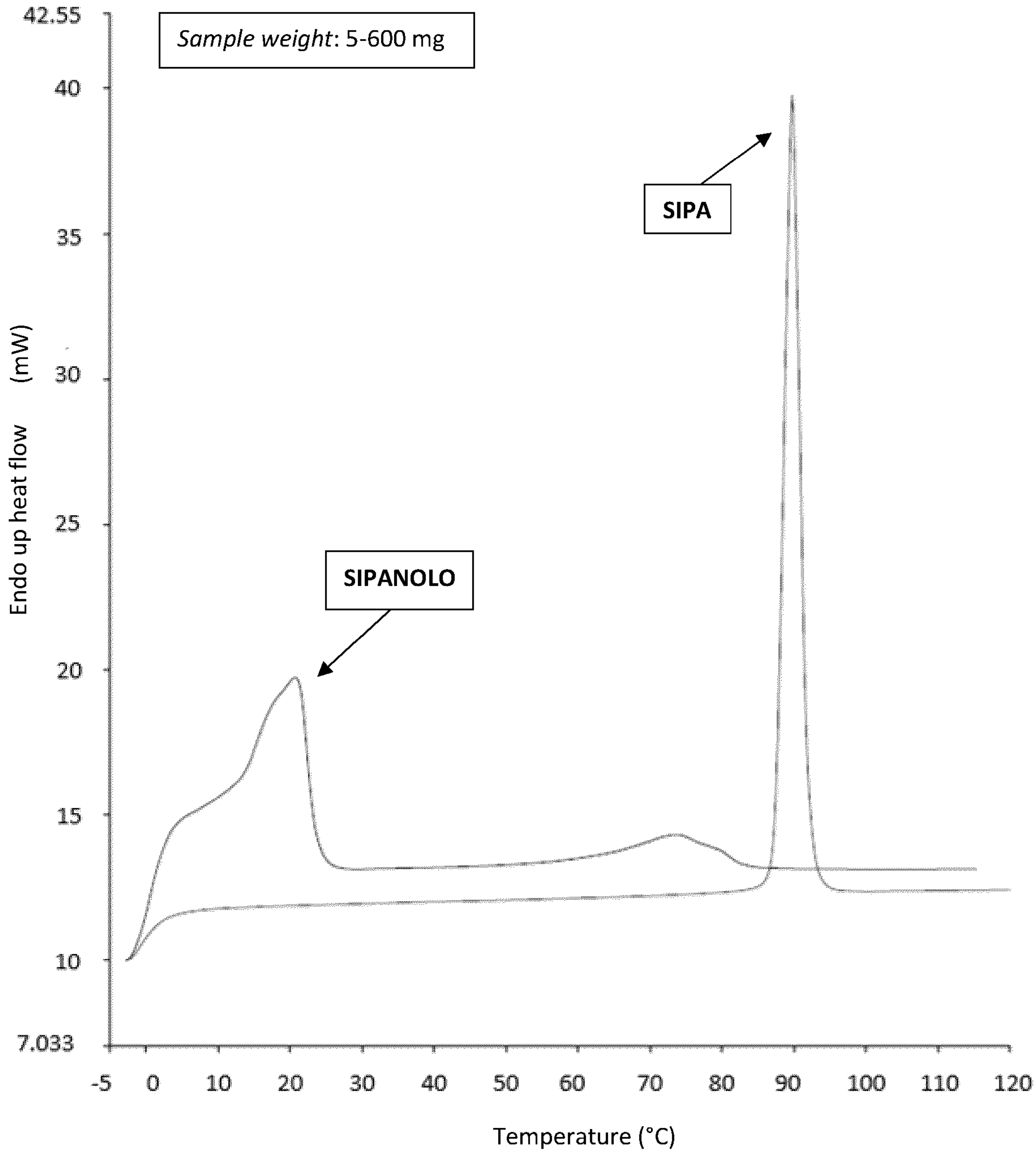
Sample weight: 5-600 mg
SIPA
SIPANOLO
Endo up heat flow (mW)
42.55
40
35
30
25
20
15
10
7.033
-5
0
10
20
30
40
50
60
70
80
90
100
110
120
Temperature (°C)

Figure 4

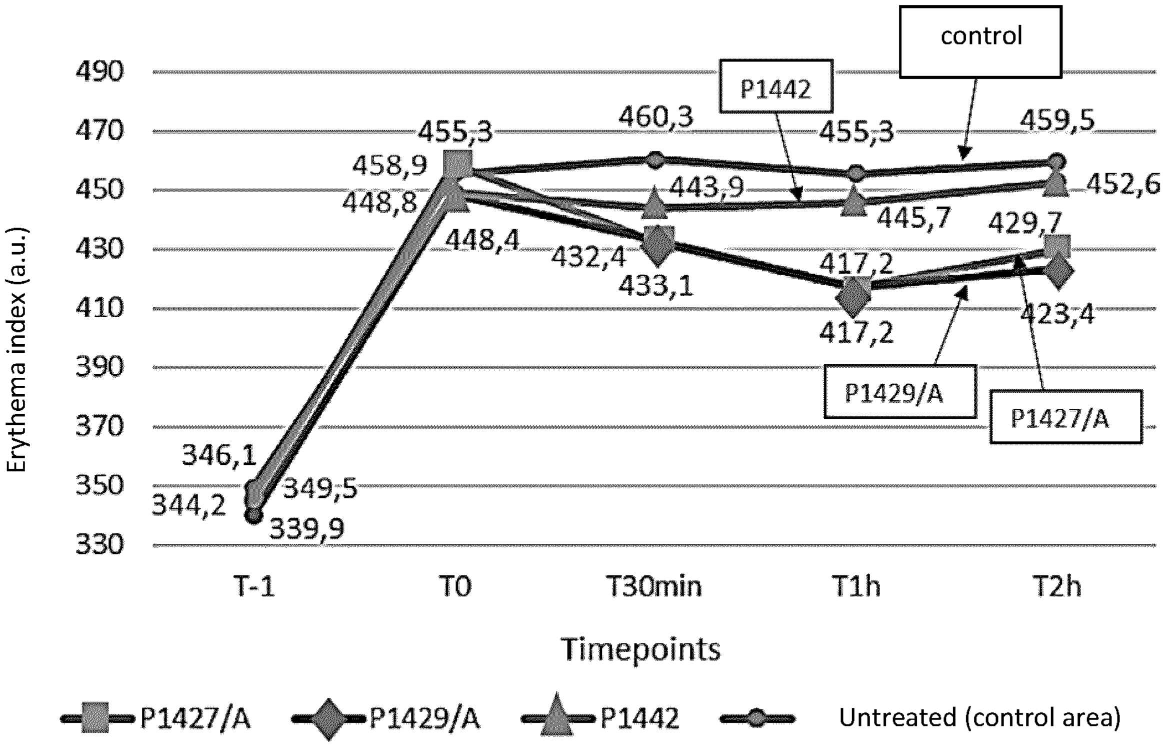


Figure 5

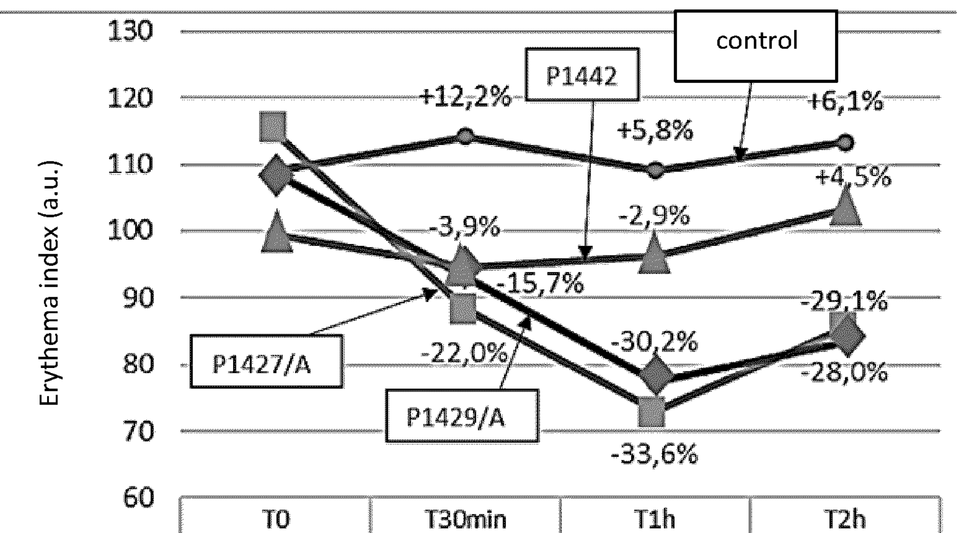


| | T0 | T30min | T1h | T2h |
|---|---|---|---|---|
| P1427/A | 114,7 | 88,3 | 73,1 | 85,5 |
| P1429/A | 108,5 | 93,2 | 77,3 | 83,5 |
| P1442 | 99,3 | 94,5 | 96,3 | 103,2 |
| Untreated (control area) | 109,2 | 114,2 | 109,2 | 113,4 |

Figure 6

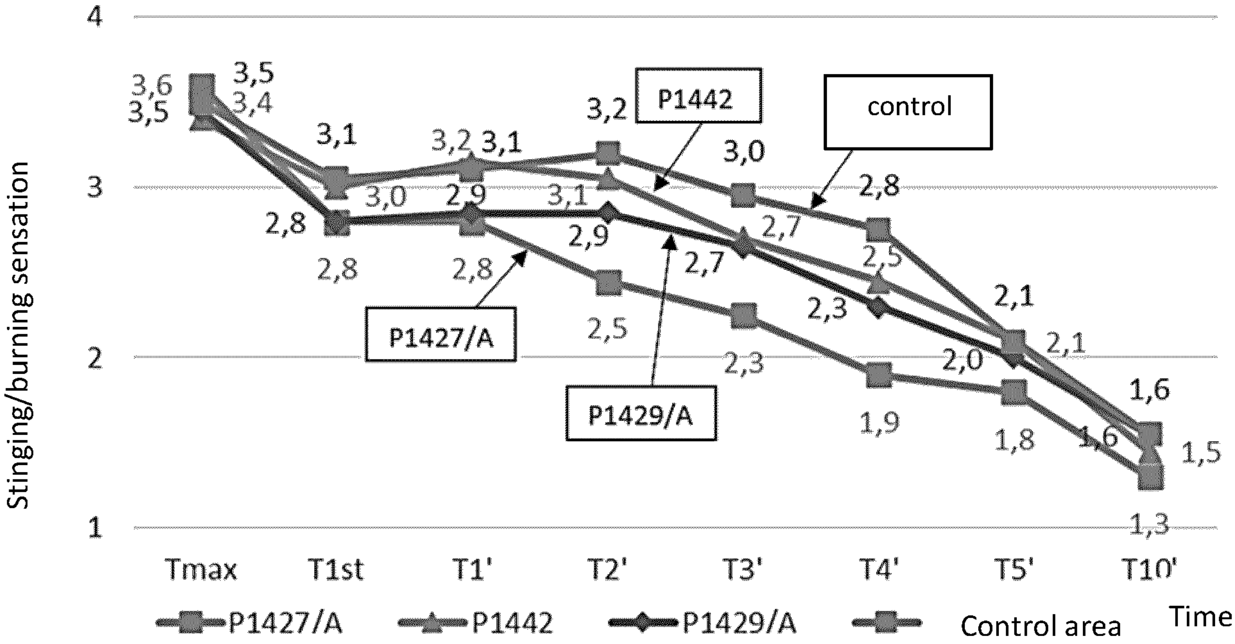

4
3
2
1
Stinging/burning sensation
3,6
3,5
3,5
3,4
3,1
3,0
2,8
2,8
3,2
3,1
2,9
2,8
3,2
3,1
2,9
2,5
P1442
3,0
2,7
2,7
2,3
control
2,8
2,5
2,3
1,9
2,1
2,1
2,0
1,8
1,6
1,6
1,5
1,3
P1427/A
P1429/A
Tmax
T1st
T1'
T2'
T3'
T4'
T5'
T10'
Time
P1427/A
P1442
P1429/A
Control area


Figure 7

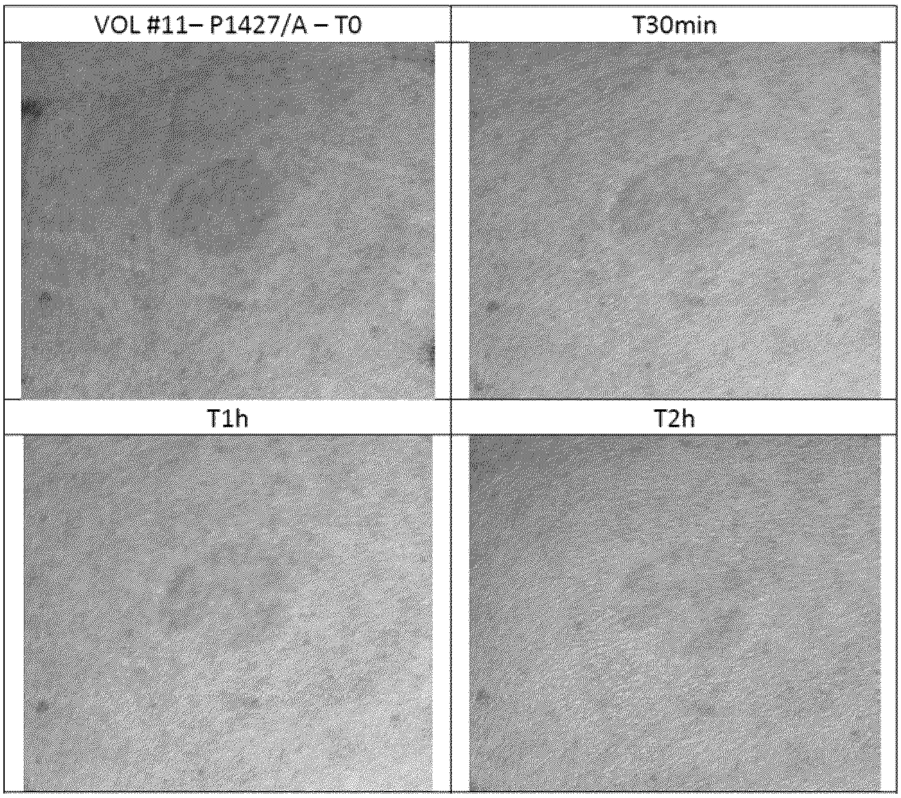


Figure 8

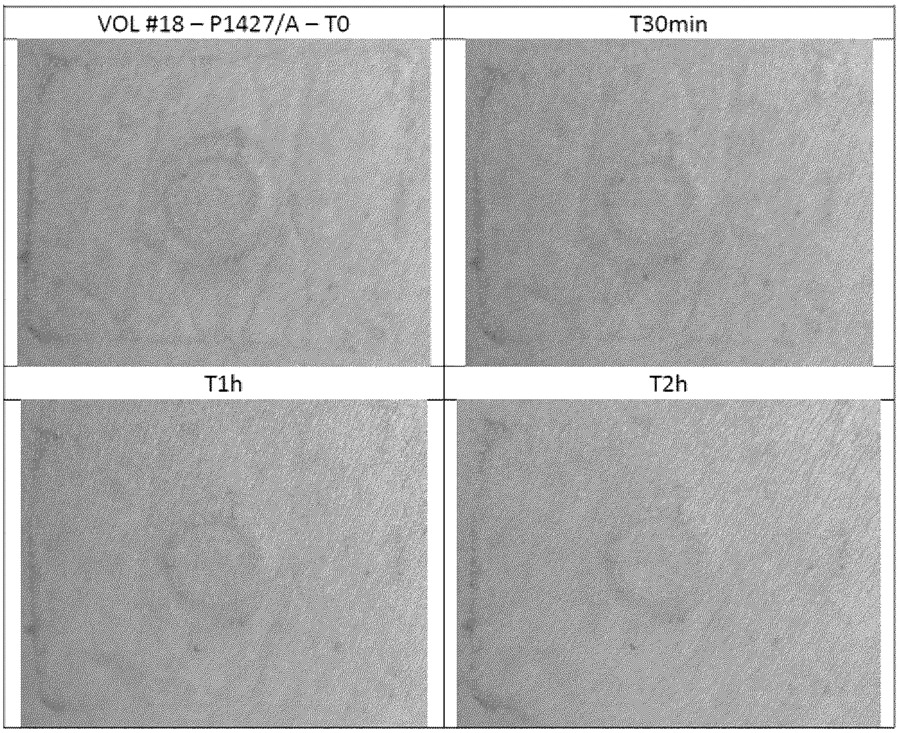


Figure 9

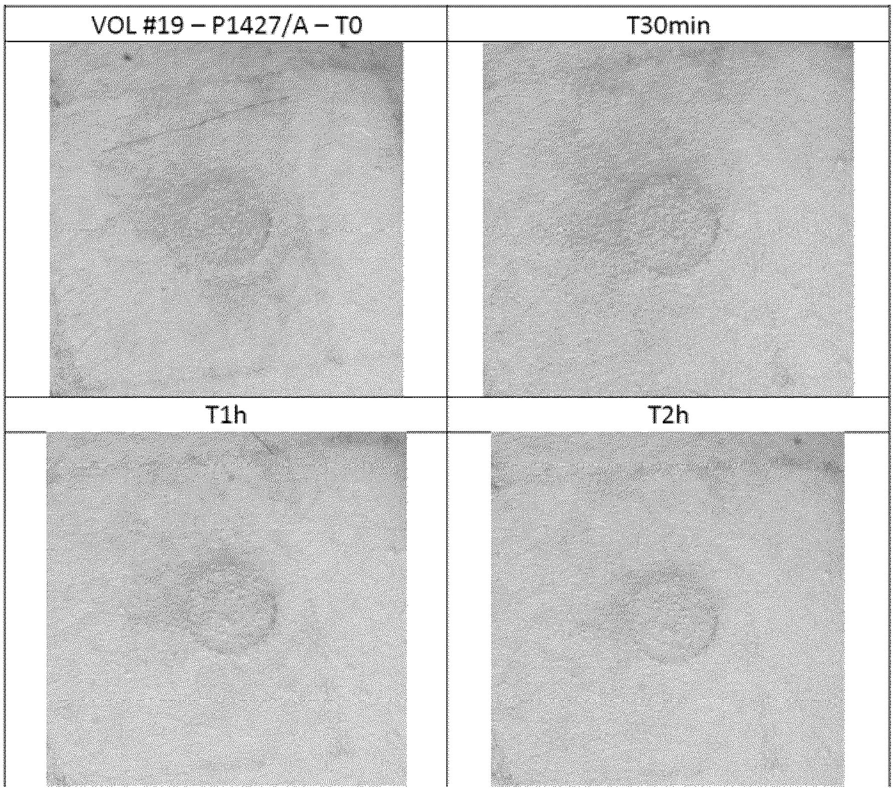


Figure 10

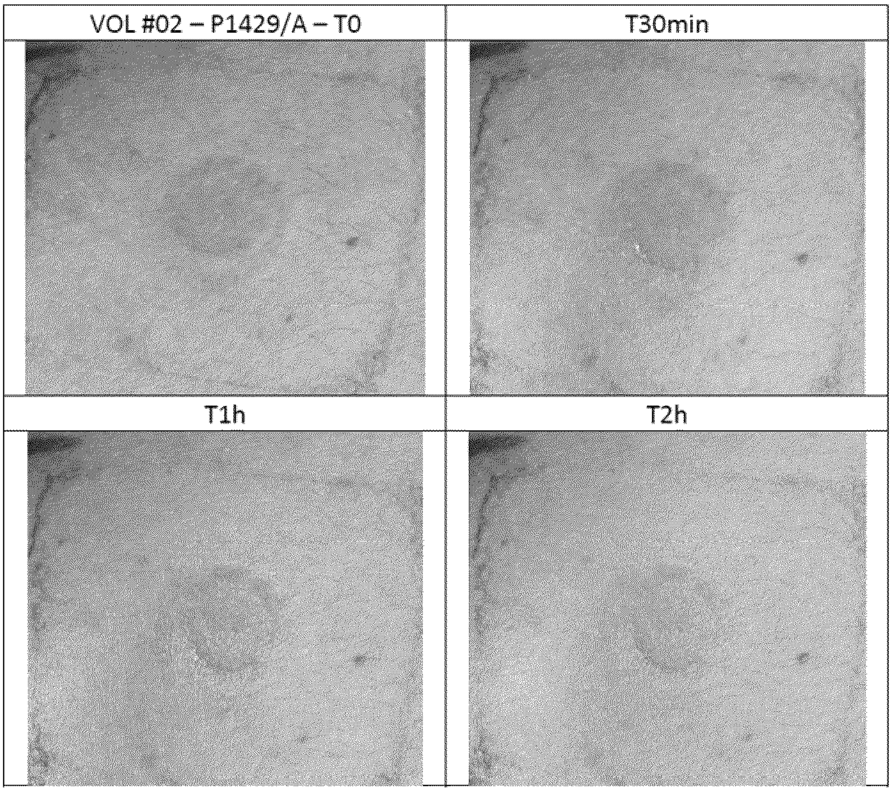


Figure 11

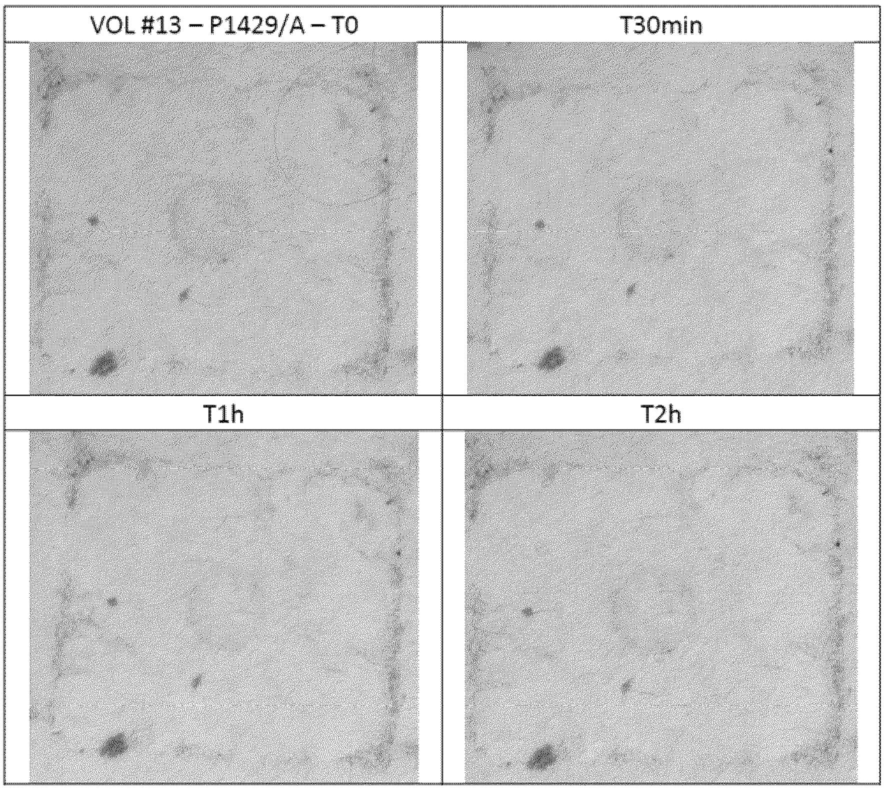


Figure 12

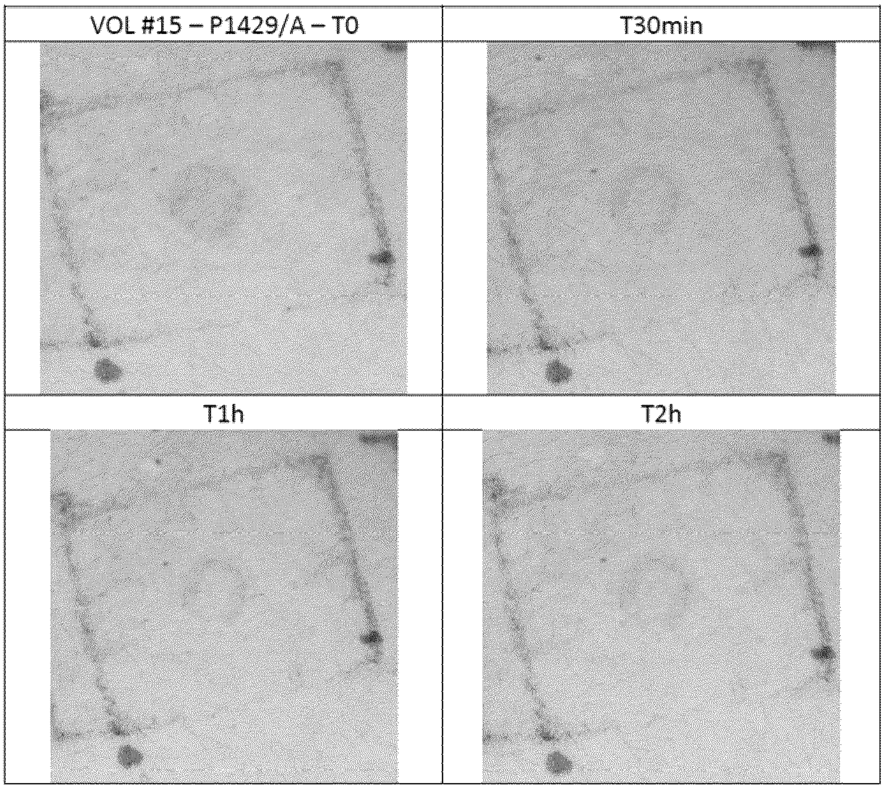


Figure 13

Europäisches Patentamt
European Patent Office
Office européen des brevets

# EUROPEAN SEARCH REPORT

Application Number
EP 25 17 4264

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | IT PD20 090 360 A1 (VERMONT ITALIA SRL) 2 June 2011 (2011-06-02) * claims 1,3, 9,10, 13 * ----- | 1-10 | INV. A61K9/00 A61K9/107 A61K31/00 A61P17/04 A61P17/06 A61K47/10 |
| Y | CN 116 983 312 A (LINGKE PHARMACEUTICAL HANGZHOU CO LTD) 3 November 2023 (2023-11-03) * the abstract describing the preparation method * ----- | 1-10 | |
| | | | TECHNICAL FIELDS SEARCHED (IPC) |
| | | | A61K A61P |

The present search report has been drawn up for all claims

2

| Place of search | Date of completion of the search | Examiner |
|---|---|---|
| The Hague | 28 July 2025 | Friederich, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 17 4264

This annex lists the patent family members relating to the patent documents cited in the above-mentioned European search report. The members are as contained in the European Patent Office EDP file on
The European Patent Office is in no way liable for these particulars which are merely given for the purpose of information.

28-07-2025

| Patent document cited in search report | Publication date | Patent family member(s) | Publication date |
|---|---|---|---|
| IT PD20090360 A1 | 02-06-2011 | ---------------------------- | |
| CN 116983312 A | 03-11-2023 | CN 116983312 A | 03-11-2023 |
| | | WO 2024239469 A1 | 28-11-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1900365 A2

### Non-patent literature cited in the description


- **LANG-ILLIEVICH K** ; **KLIVINYI C** ; **LASSER C** ; **BRENNA CTA** ; **SZILAGYI IS** ; **BORNEMANN-CIMENTI H.** Palmitoylethanolamide in the Treatment of Chronic Pain: A Systematic Review and Meta-Analysis of Double-Blind Randomized Controlled Trials.. *Nutrients*, 10 March 2023, vol. 15 (6), 1350
- **OREFICE NS** ; **ALHOUAYEK M** ; **CAROTENUTO A** ; **MONTELLA S** ; **BARBATO F** ; **COMELLI A** ; **CALIGNANO A** ; **MUCCIOLI GG** ; **OREFICE G.** Oral Palmitoylethanolamide Treatment Is Associated with Reduced Cutaneous Adverse Effects of Interferon-β1a and Circulating Proinflammatory Cytokines in Relapsing-Remitting Multiple Sclerosis. *Neurotherapeutics*, April 2016, vol. 13 (2), 428-38
- **DAVIS MP** ; **BEHM B** ; **MEHTA Z** ; **FERNANDEZ C**. The Potential Benefits of Palmitoylethanolamide in Palliation: A Qualitative Systematic Review.. *American Journal of Hospice and Palliative Medicine*, 2019, vol. 36 (12), 1134-1154
- **ALHOUAYEK M** ; **MUCCIOLI GG.** Harnessing the anti-inflammatory potential of palmitoylethanolamide.. *Drug Discov Today*, October 2014, vol. 19 (10), 1632-9
- **HOAREAU L** ; **BUYSE M** ; **FESTY F** ; **RAVANAN P** ; **GONTHIER MP** ; **MATIAS I** ; **PETROSINO S** ; **TALLET F** ; **D'HELLENCOURT CL** ; **CESARI M**. Anti-inflammatory effect of palmitoylethanolamide on human adipocytes.. *Obesity (Silver Spring)*, March 2009, vol. 17 (3), 431-8
- **EVANS M et al.** Polidocanol Inhibits Voltage-Gated Sodium Currents.. *Clin Dermatol Res J*, vol. 5
- **SOM I** ; **BHATIA K** ; **YASIR M.** Status of surfactants as penetration enhancers in transdermal drug delivery. *J Pharm Bioallied Sci.*, January 2012, vol. 4 (1), 2-9